(19) Europäisches Patentamt
European Patent Office
Office européen des brevets

(11) **EP 4 292 507 A1**

(12) **EUROPEAN PATENT APPLICATION**

(43) Date of publication:
**20.12.2023 Bulletin 2023/51**

(21) Application number: **23177902.6**

(22) Date of filing: **07.06.2023**

(51) International Patent Classification (IPC):
**A61B 1/00** (2006.01)  **A61B 1/04** (2006.01)
**A61B 1/06** (2006.01)  **A61B 1/31** (2006.01)

(52) Cooperative Patent Classification (CPC):
**A61B 1/31; A61B 1/00009; A61B 1/041;
A61B 1/0605**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.06.2022 US 202217841524**

(71) Applicant: **CapsoVision, Inc.
Saratoga, CA 95070 (US)**

(72) Inventors:
• **Wang, Kang-Huai
Saratoga, California, 95070 (US)**
• **LU, Ganyu
Palo Alto, California, 94306 (US)**

(74) Representative: **Papula Oy
P.O. Box 981
00101 Helsinki (FI)**

(54) **METHOD AND APPARATUS FOR IDENTIFYING CAPSULE CAMERA LOCATION INSIDE GASTROINTESTINAL TRACT**

(57) A method and system for identifying a GI (gastrointestinal) part associated with a location of a capsule device within a human GI tract are disclosed. According to this method, one or more structured-light images captured by the capsule device when the capsule device is inside the human GI tract are received, where the structured-light images are captured by projecting a plurality of structured light beams from the capsule device onto a surrounding lumen wall. Distance information is derived based on the structured-light images for a target set of measuring rays from the capsule device to the surrounding lumen wall. The GI part associated with the location of the capsule device associated with a distance-measuring frame related to said one or more structured-light images is determined based on the distance information, where the GI part belongs to a group comprising a transverse colon.

*Fig. 1*

## Description

### CROSS REFERENCE TO RELATED APPLICATIONS

The present invention is related to U.S. Patent No. 7,817,354 issued on Oct. 19, 2010, US Patent No. 7,983,458, issued on Jul. 19, 2011, U.S. Patent No. 9,936,151, issued on Apr. 3, 2018, U.S. Patent No. 10,506,921 issued on Dec. 17, 2019 and U.S. Patent No 11,019,327, issued on May 25, 2021. The U.S. Patents are hereby incorporated by reference in their entireties.

### FIELD OF THE INVENTION

The present invention relates to imaging the gastrointestinal (GI) tract and processing the images captured using a capsule camera. In particular, the present invention is focused on determining, based on structured-light images captured by the capsule camera, the GI anatomical part associated with the images captured by the capsule camera.

### BACKGROUND AND RELATED ART

Devices for imaging body cavities or passages *in vivo* are known in the art and include endoscopes and autonomous encapsulated cameras. Endoscopes are flexible or rigid tubes that pass into the body through an orifice or surgical opening, typically into the esophagus via the mouth or into the colon via the rectum. An image is formed at the distal end using a lens and transmitted to the proximal end, outside the body, either by a lens-relay system or by a coherent fiber-optic bundle. A conceptually similar instrument might record an image electronically at the distal end, for example using a CCD or CMOS array, and transfer the image data as an electrical signal to the proximal end through a cable. Endoscopes allow a physician control over the field of view and are well-accepted diagnostic tools. However, they do have a number of limitations, present risks to the patient, are invasive and uncomfortable for the patient, and their cost restricts their application as routine health-screening tools.

Because of the difficulty traversing a convoluted passage, endoscopes cannot easily reach the majority of the small intestine and special techniques and precautions, that add cost, are required to reach the entirety of the colon. Endoscopic risks include the possible perforation of the bodily organs traversed and complications arising from anesthesia. Moreover, a trade-off must be made between patient pain during the procedure and the health risks and post-procedural down time associated with anesthesia.

An alternative *in vivo* image sensor that addresses many of these problems is the capsule endoscope. A camera is housed in a swallowable capsule, along with a radio transmitter for transmitting data, primarily comprising images recorded by the digital camera, to a base-station receiver or transceiver and data recorder outside the body. The capsule may also include a radio receiver for receiving instructions or other data from a base-station transmitter. Instead of radio-frequency transmission, lower-frequency electromagnetic signals may be used. Power may be supplied inductively from an external inductor to an internal inductor within the capsule or from a battery within the capsule.

An autonomous capsule camera system with on-board data storage was disclosed in the US Patent No. 7,983,458, entitled "In Vivo Autonomous Camera with On-Board Data Storage or Digital Wireless Transmission in Regulatory Approved Band," granted on July 19, 2011. This patent describes a capsule system using on-board storage such as semiconductor nonvolatile archival memory to store captured images. After the capsule passes from the body, it is retrieved. Capsule housing is opened and the images stored are transferred to a computer workstation for storage and analysis. For capsule images either received through wireless transmission or retrieved from on-board storage, the images will have to be displayed and examined by diagnostician to identify potential anomalies.

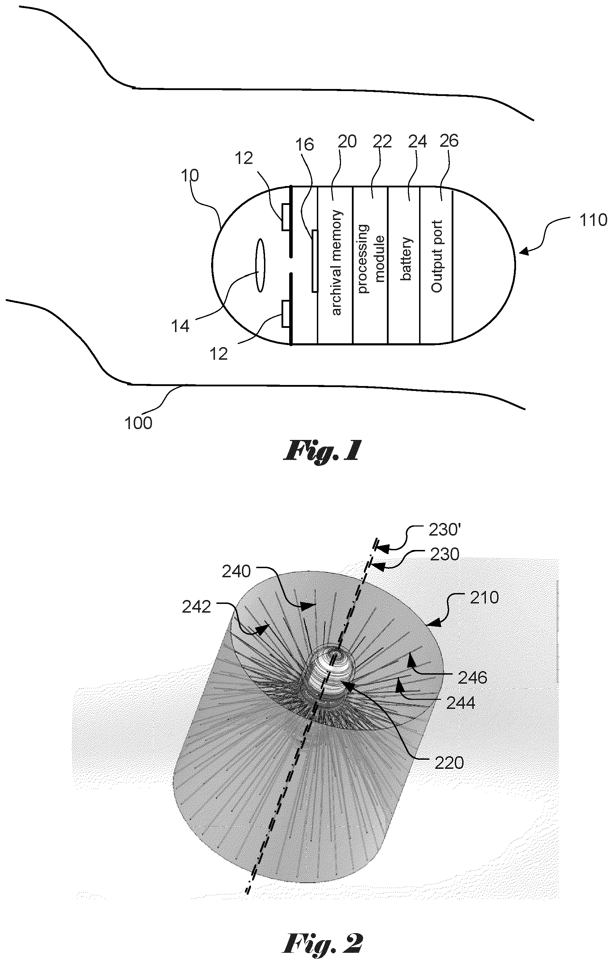

Fig. 1 illustrates an exemplary capsule system with on-board storage. The capsule system 110 includes illuminating system 12 and a camera that includes optical system 14 and image sensor 16. A semiconductor nonvolatile archival memory 20 may be provided to allow the images to be stored and later retrieved at a docking station outside the body, after the capsule is recovered. System 110 includes battery power supply 24 and an output port 26. Capsule system 110 may be propelled through the GI tract by peristalsis.

Illuminating system 12 may be implemented by LEDs. In Figure 1, the LEDs are located adjacent to the camera's aperture, although other configurations are possible. The light source may also be provided, for example, behind the aperture. Other light sources, such as laser diodes, may also be used. Alternatively, white light sources or a combination of two or more narrow-wavelength-band sources may also be used. White LEDs are available that may include a blue LED or a violet LED, along with phosphorescent materials that are excited by the LED light to emit light at longer wavelengths. The portion of capsule housing 10 that allows light to pass through may be made from bio-compatible glass or polymer.

Optical system 14, which may include multiple refractive, diffractive, or reflective lens elements, provides an image of the lumen walls on image sensor 16. Image sensor 16 may be provided by charged-coupled devices (CCD) or complementary metal-oxide-semiconductor (CMOS) type devices that convert the received light intensities into corresponding electrical signals. Image sensor 16 may have a monochromatic response or include a color filter array such that a color image may be captured (e.g. using the RGB or CYM representations). The analog signals from image sensor 16 are preferably converted into digital form to allow processing in digital form. Such conversion may be accomplished using an analog-to-digital (A/D) converter, which may be provided inside the sensor (as in the current case), or in another portion inside capsule housing 10. The A/D unit may be provided between image sensor 16 and the rest of the system. LEDs in illuminating system 12 are synchronized with the operations of image sensor 16. Processing module 22 may be used to provide processing required for the system such as image processing and video compression. The processing module may also provide needed system control such as to control the LEDs during image capture operation. The processing module may also be responsible for other functions such as managing image capture and coordinating image retrieval.

The capsule camera in Fig. 1 corresponds to an end-facing capsule, where the camera is located at the proximity of one capsule end and has a field of view along the longitudinal direction of the capsule. A capsule camera with a panoramic view has been disclosed in U.S. Patent No. 7,817,354 issued on Oct. 19, 2010, where the capsule has multiple cameras facing in directions perpendicular to the longitudinal direction of the capsule. Panoramic imaging as described in U.S. Patent No. 7,817,354 has the benefit of viewing mucosa squarely rather than mostly looking in lumen tunnel view as in end-facing cameras.

In a colonoscopy procedure, the lumen is insufflated and the view are most through air as the media. The appearance of the same pathology (e.g., a polyp) may be different from the one captured by a capsule camera since the colon is not insufflated by air. Instead, the camera may look at the polyp through liquid. Often there is a need to match the polyp from the capsule endoscopy with the polyp seen in the colonoscopy. The capsule endoscopy is a convenient alternative to the colonoscopy. However, if any anomaly (e.g., a polyp) is found during the imaging procedure by the capsule endoscopy, the anomaly has to be treated by a subsequent colonoscopy procedure. Accordingly, the location information of the polyp becomes helpful to match the polyp from the capsule endoscopy with the polyp seen in the colonoscopy. For example, if a polyp seen in the capsule endoscopy and a polyp seen in the colonoscopy are found to be from different anatomical parts of the colon (e.g., one from transverse colon and the other from the descending colon), these two polyps are apparently not the same one. Accordingly, a method to determine the anatomical parts of the colon associated with captured images are disclosed in the present invention.

## BRIEF SUMMARY OF THE INVENTION

A method and system for identifying a GI (gastrointestinal) anatomical part associated with a location of a capsule device within a human GI tract are disclosed. According to this method, one or more structured-light images captured by the capsule device when the capsule device is inside the human GI tract are received, where the structured-light images are captured by projecting a plurality of structured light beams from the capsule device onto a surrounding lumen wall. Distance information is derived based on the structured-light images for a target set of measuring rays from the capsule device to the surrounding lumen wall. Information regarding the GI anatomical part associated with the location of the capsule device associated with a distance-measuring frame related to said one or more structured-light images is provided based on the distance information, where the GI anatomical part belongs to a group comprising a transverse colon.

In one embodiment, the target set of measuring rays are on a plane perpendicular to a longitudinal axis of the capsule device and each of the measuring rays ends at an intersection of the plane and an interior point of the surrounding lumen wall. The present invention may further comprise a step of displaying visual representation of ending points of the measuring rays in a Cartesian coordinate on a display device.

In one embodiment, the present invention may further comprise a step of providing information indicative of a shape of the surrounding lumen wall based on the distance information, where the location of the capsule device is indicated according to whether the shape of the surrounding lumen wall is closer to a circle or a triangle. In one embodiment, curve fitting is used to determine whether the shape of the surrounding lumen wall resembles the triangle.

In one embodiment, the method further comprises displaying a plot on a display device for a user to visualize, where the plot is representative of the shape of the surrounding lumen wall in one axis and a frame number or a frame time in another axis. The GI anatomical part associated with the location of the capsule device is indicated according to the shape of the surrounding lumen wall, where if the shape resembles the triangle, the GI anatomical part associated with the location of the capsule device is the transverse colon.

In one embodiment, the method further comprises displaying a plot on a display device for a user to visualize, where the plot is representative of a shape index of the surrounding lumen wall in one axis and a frame number or a frame time in another axis, and wherein the shape index is derived based on frequency-domain data of the distance information represented in a polar coordinate. The GI anatomical part associated with the location of the capsule device is indicated according to the shape index and if the shape index indicates the triangle, the GI anatomical part associated with the location of the capsule device is the transverse colon.

In one embodiment, the present invention may further comprise a step of determining a size of the surrounding lumen wall based on the distance information. A plot can be displayed on a display device, where the plot is representative of the size of the surrounding lumen wall in one axis and the frame number or the frame time in another axis, where the location of the capsule device is indicated according to the size of the surrounding lumen wall. A section of the human tract corresponds to an ascending colon if the section corresponds to a first part of the human colon and the size is reduced or the size is gradually reducing. In another embodiment, the size of the surrounding lumen wall is indicated based on the distance information associated with multiple distance-measuring frames close to each other in a temporal order. The GI anatomical part associated with the location of the capsule device is determined based on the distance information associated with the multiple distance-measuring frames, and a decision of the GI anatomical part associated with the location of the capsule device is confirmed when a same decision out of multiple consecutive decisions is reached. In yet another embodiment, the plot representative of the size of the surrounding lumen wall corresponds an average or filtered size of the surrounding lumen wall associated with multiple measuring frames

In one embodiment, the present invention may further comprise a step of transforming the distance information represented in a polar coordinate into discrete frequency-domain information, where the GI anatomical part associated with the location of the capsule device is indicated by the discrete frequency-domain information. For example, Discrete Fourier Transform (DFT) or Fast Fourier Transform (FFT) can be used to transform the distance information into the discrete frequency-domain information. For example, if all frequency terms are insignificant except for zero-th frequency term, the GI anatomical part associated with the location of the capsule device corresponds to ascending or descending colon. In another embodiment, if the discrete frequency-domain information has a maximum at zero-th frequency term of the discrete frequency-domain information and a second largest term at third frequency term of the discrete frequency-domain information, the GI anatomical part associated with the location of the capsule device corresponds to the transverse colon. In one embodiment, the present invention may further comprise a step of determining a magnitude ratio of third frequency term and zero-th frequency term of the discrete frequency-domain information, where if the magnitude ratio is larger than a threshold, the GI anatomical part associated with the location of the capsule device corresponds to the transverse colon and, otherwise the GI anatomical part associated with the location of the capsule device corresponds to the ascending/descending colon. In one example, the threshold includes a range between 0.13 and 0.27.

In one embodiment, the present invention may further comprise applying correction to the distance information prior to transforming the distance information into the discrete frequency-domain information. For example, coordinate translation can be applied to the distance information to correct eccentricity. In another example, coordinate rotation can be applied to the distance information to correct tilting. In yet another example, coordinate rotation followed by coordinate translation are applied to the distance information to correct tilting and eccentricity respectively. In yet another example, coordinate translation followed by coordinate rotation are applied to the distance information to correct eccentricity and tilting respectively.

In one embodiment, the present invention may further comprise a step of receiving a regular image capsuled by a camera in the capsule device for a scene in a field view of the camera and determining an anomaly in the regular image, where the regular image is temporally close to said one or more structured-light images and the GI anatomical part associated with the location of the anomaly within the human GI tract is determined based on the distance information.

In one embodiment, the present invention may further comprise a step of determining fold depth information of the surrounding lumen wall based on the distance information. Furthermore, a plot can be displayed on a display device for a user to visualize, where the plot is representative of the fold depth information of the surrounding lumen wall in one axis and a frame number or a frame time in another axis. the GI anatomical part associated with the location of the anomaly, within the human GI tract is indicated based on the fold depth information.

In another embodiment, the method further comprises displaying a plot on a display device for a user to visualize, wherein the plot is representative of the discrete frequency-domain information in one axis and a frame number or a frame time in another axis. For example, the discrete frequency-domain information corresponds to a ratio of a magnitude of third frequency term to a magnitude of zero-th frequency term.

## BRIEF DESCRIPTION OF THE DRAWINGS

Fig. 1 illustrates an exemplary capsule system with on-board storage, where the capsule system includes illuminating system and a camera that includes optical system and image sensor.

Fig. 2 illustrates an example of structured lights for measuring distances from a capsule camera to surrounding lumen walls.

Fig. 3 illustrates an example of structured lights for measuring distances, where the flat measuring cone plane passing through the capsule.

Fig. 4 illustrates another example of structured lights for measuring distances, where the measuring cone is at a forward location of the capsule.

Fig. 5A illustrates an example of the distances of the target set of rays for the case of no eccentricity and no tilting.

Fig. 5B represents a plot of the measured distances versus the polar angle, i.e., distances in the polar coordinate.

Fig. 5C illustrates an example of the distances of the target set of rays for the case of eccentricity and no tilting.

Fig. 5D represents a plot of the measured distances versus the polar angle, i.e., distances in the polar coordinate.

Fig. 6A illustrates an example of distances from the capsule to the lumen wall for the transverse colon with ideal triangular wrinkles or folds the capsule centered and without tilting.

Fig. 6B illustrates the distances in the polar coordinate for the example of Fig. 6A.

Fig. 6C illustrates another example of distances from the capsule to the lumen wall for the transverse colon with ideal triangular wrinkles or folds and with the capsule centered and without tilting.

Fig. 6D illustrates the distances in the polar coordinate for the example of Fig. 6B.

Fig. 6E illustrates an example of distances from the capsule to the lumen wall for the transverse colon similar to Fig. 6A, but with eccentricity.

Fig. 6F illustrates the distances in the polar coordinate for the example of Fig. 6E.

Figs. 7A-B illustrate examples of measured lumen size profile for the ascending colon during a course of imaging the colon by a capsule device.

Figs. 8A-E illustrate the amplitudes of the lower frequency terms of the frequency response for the centered circle case of Fig. 5A, the circle with eccentricity case of Fig. 5C, the centered triangle case of Fig. 6A, the centered triangle with sharper corners case of Fig. 6C, and the triangle with eccentricity case of Fig. 6E respectively.

Fig. 9A illustrates an example of distances from the capsule to the circular lumen wall with tilting, where the capsule is tilted by about 60° from the longitudinal direction of the GI tract.

Fig. 9B shows an example of tilting with eccentricity, where the capsule is tilted by about 60° from the longitudinal direction of the GI tract and 23mm off center.

Fig. 9C illustrates the distances in the polar coordinate for Fig. 9A.

Fig. 9D illustrates the distances in the polar coordinate for Fig. 9B.

Fig. 9E illustrates the amplitude of the lower frequency terms of the frequency response for Fig. 9A.

Fig. 9F illustrates the amplitude of the lower frequency terms of the frequency response for Fig. 9B.

Fig. 10A illustrates an example of distances from the capsule to the triangular lumen wall with tilting, where the capsule is tilted by about 60° from the longitudinal direction of the GI tract.

Fig. 10B shows an example of tilting with eccentricity, where the capsule is tilted by about 60° from the longitudinal direction of the GI tract and 19mm off center.

Fig. 10C illustrates the distances in the polar coordinate for Fig. 10A.

Fig. 10D illustrates the distances in the polar coordinate for Fig. 10B.

Fig. 10E illustrates the amplitude of the lower frequency terms of the frequency response for Fig. 10A.

Fig. 10F illustrates the amplitude of the lower frequency terms of the frequency response for Fig. 10B.

Fig. 11A illustrates an example of distances from the capsule to the lumen wall of the transverse colon in an in-vitro environment, where the capsule is off center without tilting.

Fig. 11B illustrates the corresponding distances in the polar coordinate for Fig. 11A.

Fig. 11C illustrates the distances for Fig. 11A after eccentricity correction according to an embodiment of the present invention.

Fig. 11D illustrates the distances in the polar coordinate for Fig. 11C.

Fig. 11E illustrates the amplitude of the lower frequency terms of the frequency response for Fig. 11A.

Fig. 11F illustrates the amplitude of the lower frequency terms of the frequency response for Fig. 11C.

Figs. 12A-B illustrate examples of shape index of measured lumen shape profile for the colon during a course of imaging the colon by a capsule device.

Fig. 13 illustrates an exemplary flowchart for identifying a GI (gastrointestinal) anatomical part associated with a location of a capsule device within a human GI tract according to an embodiment of the present invention.

## DETAILED DESCRIPTION OF THE INVENTION

It will be readily understood that the components of the present invention, as generally described and illustrated in the figures herein, may be arranged and designed in a wide variety of different configurations. Thus, the following more detailed description of the embodiments of the systems and methods of the present invention, as represented in the figures, is not intended to limit the scope of the invention, as claimed, but is merely representative of selected embodiments of the invention. References throughout this specification to "one embodiment," "an embodiment," or similar language mean that a particular feature, structure, or characteristic described in connection with the embodiment may be included in at least one embodiment of the present invention. Thus, appearances of the phrases "in one embodiment" or "in an embodiment" in various places throughout this specification are not necessarily all referring to the same embodiment.

Furthermore, the described features, structures, or characteristics may be combined in any suitable manner in one or more embodiments. One skilled in the relevant art will recognize, however, that the invention can be practiced without one or more of the specific details, or with other methods, components, etc. In other instances, well-known structures, or operations are not shown or described in detail to avoid obscuring aspects of the invention. The illustrated embodiments of the invention will be best understood by reference to the drawings, wherein like parts are designated by like numerals throughout. The following description is intended only by way of example, and simply illustrates certain selected embodiments of apparatus and methods that are consistent with the invention as claimed herein.

Endoscopes are normally inserted into the human body through a natural opening such as the mouth or anus. Therefore, endoscopes are preferred to be small sizes so as to be minimally invasive. As mentioned before, endoscopes can be used for diagnosis of human gastrointestinal (GI) tract. The captured image sequence can be viewed to identify any possible anomaly. If any anomaly is found, it is of interest to identify the location of the polyp, which is useful for subsequent treatment such as removal of the polyp. Accordingly, the present invention discloses an endoscope incorporating a means for determining the location of the capsule when a target image is captured, in particular, when the target image is determined to contain a polyp.

There are various known ways to determine the location of a capsule. For example, in U.S. Patent No. 10,506,921 issued on Dec. 17, 2019, a method is disclosed to estimate the travelled distance measuring by a capsule camera in the gastrointestinal tract. Accordingly, the location of the capsule can be determined in terms of travelled distance inside the GI tract. In U.S. Patent No. 10,354,382 issued on Jul. 16, 2019, a method is disclosed to use features, such as the geometric shape of the colon in the captured images to recognize the segment of the colon. For example, the lumen of the transverse colon has a particularly triangular shape. An image of the colon's geometric shape can be compared with a database of images the colon's geometric shape to determine which segment of the colon the imaging device is in. In U.S. Patent No. 10,102,334 issued on Jul. 16, 2019, a method is disclosed to use pattern recognition based on the wrinkle patterns or wall structure of the colon to automatically detect whether the wrinkles of the tissue walls correspond to the transverse colon or ascending/descending colon.

Also, certain kinds of localization devices, such as an accelerator, gyrator, etc., have been used to trace the ingestible device in the GI tract when the ingestible capsule containing these localization devices passes through the GI tract. However, the subject usually does not remain stationary after swallowing the ingestible capsule. When the subject moves, these localization devices cannot reliably differentiate the movement of torso or the capsule in the GI tract.

In the present invention, a method to differentiate the anatomical parts of the GI tract based on structured-light images is disclosed. The capsule camera in the present invention incorporates light sources and micro-lens array to project structured light onto the surrounding lumen walls. The structured-light images are captured and used to derived depth or shape information of the corresponding regular images captured by the capsule camera. Methods and apparatus for capturing regular images and structured light images using a single image sensor are disclosed in U.S. Patent No. 9,936,151, issued on Apr. 3, 2018. In U.S. Patent No 11,019,327, issued on May 25, 2021 discloses details of the structured light source and the micro-lens array. According to U.S. Patent No. 9,936,151, the structured-light images can be used to derive depth or shape information for the corresponding regular images.

In one embodiment, structured light beams are projected onto the lumen walls and distances for the measuring rays from the capsule to the lumen wall are derived from the structured light, where a set of measuring rays from the capsule to the lumen wall are used to determine the distances from the capsule to a perimeter of the GI tract. The ending points of the measuring rays are located on the perimeter. The perimeter of the GI tract is preferred to be aligned with the field of view for regular images since the distance information will be used by the corresponding regular images. When a pathology feature, such as a polyp, is found in a regular picture, the corresponding distance may be used to determine where the polyp is located in the GI tract. The set of measuring rays may have a cone shape with the tip of cone at the capsule. In an extreme case, the cone may be fully open to become flat. In this case, the perimeter corresponds to the intersection of the GI tract and a plane perpendicular to the longitudinal axis of the capsule. The cone for the set of measuring rays may be in a forward or backward direction of the capsule.

For the purpose of illustration, we now assume the structure of the lumen wall can be modelled by a target geometry shape such as a cylinder or a round triangular prism. If a section of the GI tract is model as a cylinder, then a cross section of the lumen wall perpendicular to the longitudinal direction becomes a circle. Fig. 2 illustrates an example, where a capsule 220 projects structured light beams (e.g., 240, 242, 244 and 246) onto the lumen wall of a section of GI tract 210. The capsule is assumed to be located at the center of the lumen wall with the longitudinal axis of the capsule (shown as a dashed line 230) aligned with the longitudinal direction of the GI tract 230' (shown as a dot-dash line 230'). Various patterns of the structured light beams can be used. For imaging the GI tract, a pattern comprising layers of circular dots is disclosed in U.S. Patent No 11,019,327. Nevertheless, the present invention is not limited by any specific pattern of structured light beams. Any beam pattern that can be used to derive the distances for a target set of rays from the capsule to the intersection of the lumen wall and a measuring cone, where the measuring cone can be perpendicular to the longitudinal direction of the GI tract. When the GI tract is modelled as a cylinder, the intersection of the lumen wall and the measuring cone becomes a circle.

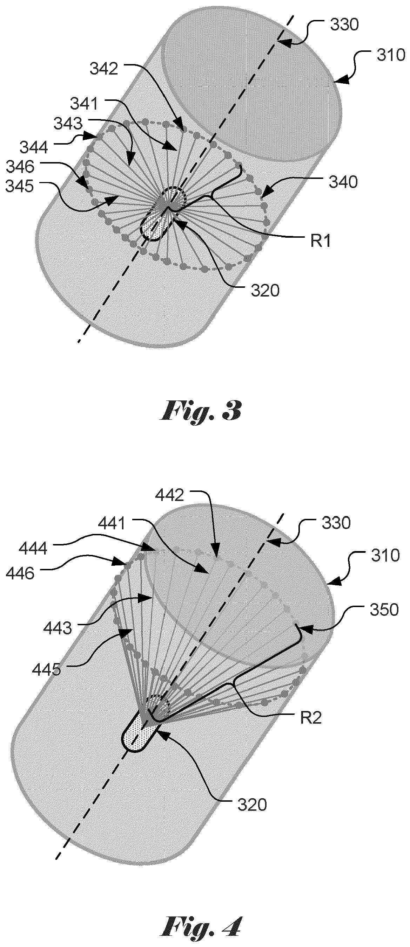

Fig. 3 illustrates an exemplary target set of measuring rays (341, 343, 345, etc.) associated with a flat measuring cone passing through the capsule 320. The intersection between the flat measuring cone and the GI wall 310 is a circle 340. It is understood that the flat measuring cone is perpendicular to the longitudinal axis 330 of the capsule (also the GI tract) and contains the intersection circle 340. The target set of rays (341, 343, 345, etc.) and the intersection points (342, 344, 346, etc.) of the rays and the GI wall are shown in Fig. 3. The distances of the rays in this case have a constant value (i.e., radius R1). Fig. 4 illustrates another example of the measuring cone, where the measuring cone is located in a forward position to the capsule. The intersection between the measuring cone and the GI wall 310 is still a circle 350. The target set of rays (441, 443, 445, etc.) and the intersection points (442, 444, 446, etc.) of the rays and the GI wall are shown in Fig. 4. The distances of the rays in this case have a constant value (i.e., radius R2), which is larger than the radius (i.e., R1) for the example in Fig. 3. For a panoramic capsule camera, the images captured by the camera will be centered around a plane perpendicular to the longitudinal axis of the camera when the longitudinal direction of the capsule is aligned with the longitudinal direction of the GI tract. Therefore, the flat measuring cone through the capsule is preferred for the panoramic camera. For an end-facing capsule, the measuring cone toward the imaging end of the capsule is preferred.

As shown in Fig. 2, the structured-light beams often cover similar field of view as the regular images. Therefore, the structured-light image may cover a large region of lumen wall around the capsule device. On the other hand, the distance information for measuring the size/shape of the lumen wall may be confined to the perimeter as shown in Fig. 3 and Fig. 4. The target set of rays for measuring the size/shape of the lumen wall may not be at the exact same locations of the structured light beams. However, as is known in the art, the distances of the target set of rays can be derived from the structured light beams.

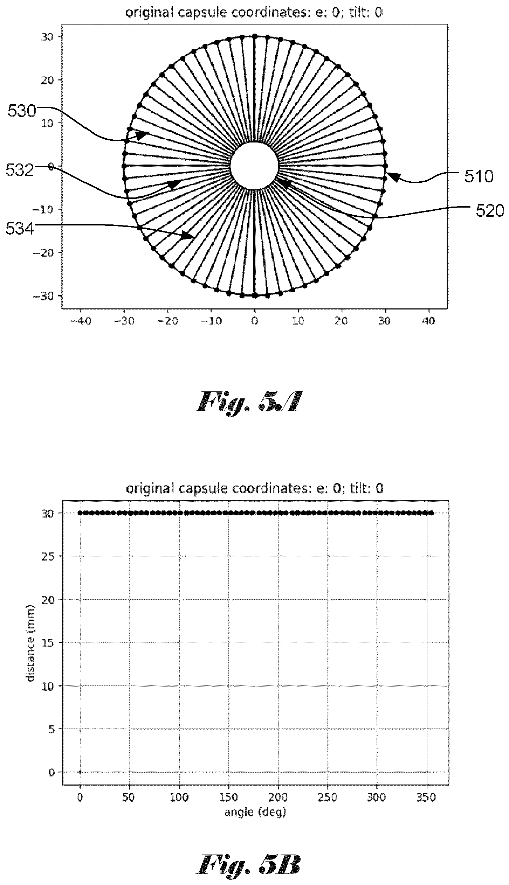

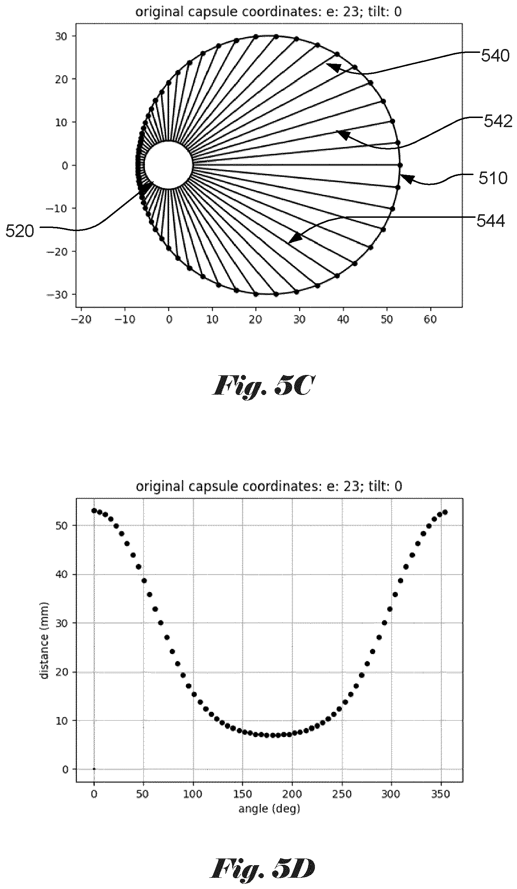

Fig. 5A illustrates an example of the distances of the target set of rays (530, 532, 534, etc.) for the case of no eccentricity (i.e., capsule 520 at the center of the GI tract 510) and no tilting (i.e., the longitudinal axis of the capsule aligned with the longitudinal direction of the GI track). The "eccentricity" is defined as the eccentricity of capsule center relative to the GI tract center. In Fig. 5A, the distances information as represented by the measuring rays from the capsule to the lumen wall are displayed in an x-y coordinate (i.e., in a Cartesian coordinate) with the center of the shape as the origin, which renders a useful visual presentation on a display device for a user (e.g., a medical professional) to visualize the size/shape of the lumen wall. In particular, the ending points of the measuring rays provide a good visual representation of the contour of the lumen wall for the size and/or shape of the lumen wall. Fig. 5B represents a plot of the measured distances versus the polar angle. In this case, all distances are the same and the plot becomes a flat line. Fig. 5C illustrates an example of the distances of the target set of rays (540, 542, 544, etc.) in the x-y coordinate with the center of the shape as the origin for the case of eccentricity (i.e., capsule 520 off the center of the GI tract 510) and no tilting (i.e., the longitudinal axis of the capsule aligned (i.e., parallel) with the longitudinal direction of the GI track). The "tilting" is defined as the tilting of capsule longitudinal axis relative to GI tract centerline. Fig. 5D represents a plot of the measured distances versus the polar angle. In this case, the distance varies along the polar angle with a maximum at 0° (or 360°) and a minimum at 180°.

In U.S. Patent No. 9,936,151, an endoscope device (tethered or capsule) is disclosed, which is capable of capturing regular images as well as structured-light images. The structured-light images are captured along with the regular images in an interleaved fashion so that each regular image is always accompanied by one or more close-by (in the temporal order) structured-light images. Therefore, the depth data extracted from the close-by structured-light images can be used for the corresponding regular image. For example, if a polyp is found in a regular image, the size information of the polyp can be estimated from the corresponding close-by structured-light images. For a capsule device, when polyp is found, the polyp, has to be removed in a subsequent colonoscopy procedure. Therefore, the location information, in terms of which part of the colon, such as ascending colon, transverse or descending colon, regarding the polyp becomes useful.

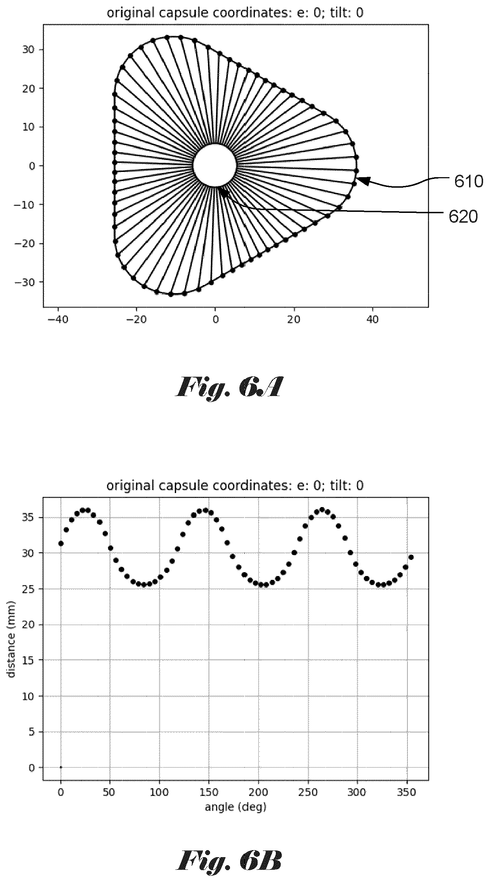

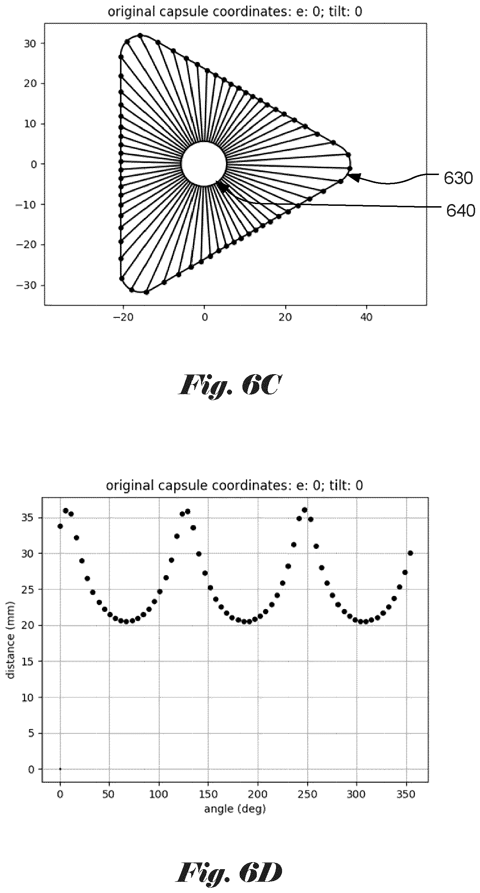

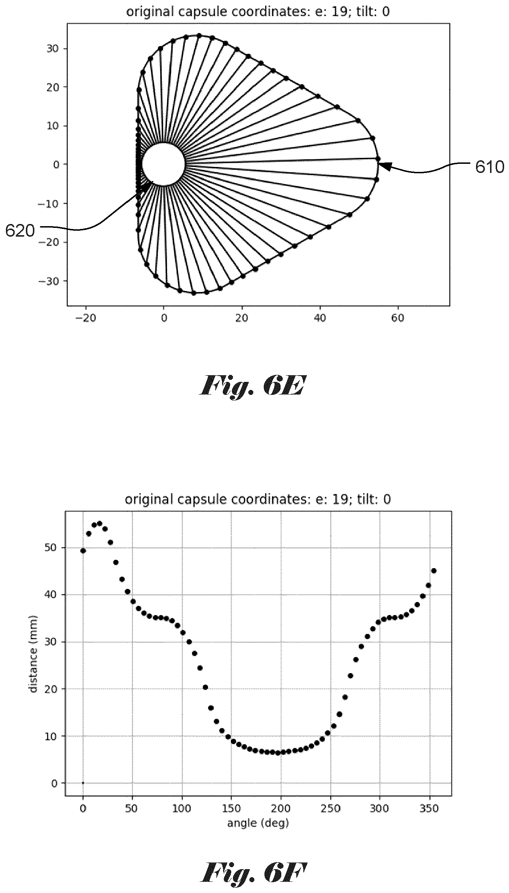

As mentioned earlier, the cylinder model fits the ascending and descending colons well. However, for the transverse colon, a triangular model fits better. As disclosed in various literatures related to the colon, where the wrinkles of the tissue walls in the transverse colon show a triangular structure. Therefore, the transverse colon can be modelled as triangular wrinkles or folds inside the GI tract. An intersection of the interior of the lumen wall and a flat measuring cone perpendicular to the longitudinal direction should have a triangular shape with round corners. Fig. 6A illustrates an example of distances from the capsule to the lumen wall for the transverse colon with ideal triangular wrinkles or folds, the capsule centered and without tilting, where triangle 610 corresponds to the intersection of the interior of the lumen wall and a flat measuring cone perpendicular to the longitudinal direction and capsule 620 is located at the center of the GI tract. The distance versus polar angle for the example of Fig. 6A is shown in Fig. 6B. As shown in Fig., 6B, the distance oscillates up and down for three cycles from 0° to 360°. Fig. 6C illustrates another example of distances from the capsule to the lumen wall for the transverse colon with ideal triangular wrinkles or folds and with the capsule centered and without tilting, where triangle 630 corresponds to the intersection of the interior of the lumen wall and a flat measuring cone perpendicular to the longitudinal direction and capsule 640 is located at the center of the GI tract. In this example, the model for the transverse colon has sharper corners. The distance versus polar angle for the example of Fig. 6C is shown in Fig. 6D. As shown in Fig., 6D, the distance oscillates up and down for three cycles from 0° to 360°. Fig. 6E illustrates an example of distances from the capsule to the lumen wall for the transverse colon similar to Fig. 6A. However, in this case, the capsule is off the center of the GI tract and however, the longitudinal axis of the capsule is still aligned (i.e., parallel) with the longitudinal direction of the GI tract. The distance versus polar angle for the example of Fig. 6E is shown in Fig. 6F.

From the planar (i.e., two-dimensional) contour plots (e.g. Figs. 5A, 5C, 6A, 6C and 6E) of the lumen wall based on the derived distances from the capsule to the lumen wall, the shape of the lumen wall (in particular, the interior wall) can be identified visually. Therefore, depending on whether the shape looks triangular or round, we can differentiate whether the capsule is in the transverse colon or the ascending/descending colon. The decision for the capsule location within the GI tract is made based on the distance information, which is derived from the structured-light images. Therefore, the capsule location determined is temporally associated with the structured-light image (referred as a frame). The distance information may also be derived from multiple structured-light images. In this case, the time associated with the capsule location can be determined based on the frame times. For example, if the distance information is derived from two frames of the structured-light images, the time associated with the capsule location can be the average of the two frame times. Alternatively, it may also designate any of the two frame times as the time associated with the capsule location. If the distance information is derived from three structured-light image frames, the time associated with the capsule location can be the center of the three frame time. In general, the center of the multiple frame times can be designated as the time associated with the capsule location. Furthermore, we may track the shape/size transition to determine when the capsule enters the transverse colon from the ascending colon or when the capsule enters the descending colon from the transverse colon.

Since there are variations among different subjects and the colon is not a uniform tract, this dependence on the size could be based on the frame number or the time along one axis and the size in another axis. Consequently, according to another embodiment of the present invention, the indication of anatomical parts of the colon associated with the measuring frames is based on the size or the trend of the size along the frame number axis or the time axis. Alternatively, it can also be based on the shape or the trend of shape or based on the distance information. For example, the shape resembles a triangular type is associated with the transverse colon and round; or otherwise with the ascending, descending, sigmoid colon or rectum. The size or the trend of the size along the frame number axis or the time axis can be displayed on a display device for a user (e.g., a medical professional) to visualize. The user can determine the anatomical parts of the colon associated with the measuring frames based on the displayed information.

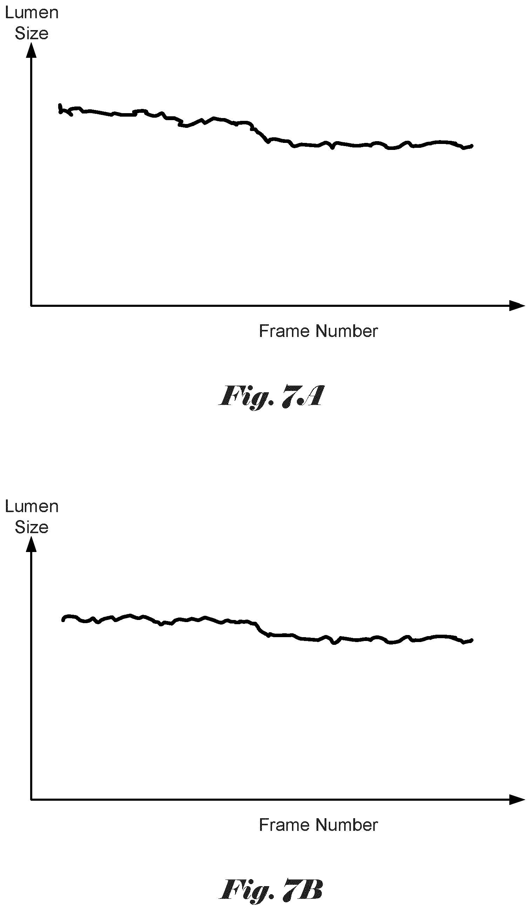

We have observed that the beginning section (closer to the cecum) of the ascending colon is usually larger than the distal section (i.e., closer to transverse colon) in size. Therefore, the distance between the capsule and the mucosal surface (i.e., the lumen wall) tends to be larger for the beginning section of the ascending colon. As mentioned earlier, the size of the lumen can be determined from the distance between the capsule and the lumen wall. Therefore, we can generate a drawing corresponding to the distance information or lumen size information versus elapse time or travel time, where the elapse time or travel time is associated with the time when the capsule travelling through the colon. The elapse time or capsule travel time can also be replaced by frame number since the frames of structured-light images are captured sequentially when the capsule travels through the GI tract. Figs. 7A-B illustrate two examples of the lumen size information versus the capsule travel time in the GI tract. As shown in Figs. 7A-B, the size profiles for two different subjects appear to be quite different. However, the lumen size profile shows a trend of reducing size along time, which corresponds to the capsule device moves from ascending colon to distal part of colon. The trend of the lumen size along the frame number axis or the time axis can be displayed on a display device for a user (e.g., a medical professional) to visualize. The user can determine the anatomical parts of the colon associated with the measuring frames based on the displayed information.

The colon fold in the ascending colon is also larger and deeper than the distal colon. By using 3D information, we can also discern the fold depth and accordingly discern whether it is in the ascending colon or not based on the fold depth or its trend along the time or frame axis. The depth of a particular fold can be estimated by the difference of the distance to the edge of the fold and the distance to the base, i.e. the surrounding mucosa next to the fold. The fold depth or the trend of the fold depth along the frame number axis or the time axis can be displayed on a display device for a user (e.g., a medical professional) to visualize. The user can determine the anatomical parts of the colon associated with the measuring frames based on the displayed information.

While the task of identifying which part of the colon that the capsule is located can be performed by a human subject, the task can also be automated, or at least partially automated, using a computing device such as a personal computer, a notebook, a workstation, a mobile device such as smart phone, or an embedded processor. As mentioned earlier, the shape of the colon fits a circular model or a triangular model depending on whether the capsule camera is located in the ascending/descending colon or the transverse colon. Therefore, we can fit the distance ending dots corresponding to the intersection of the lumen wall and a measuring cone with the two candidate shapes, i.e., a circle and a triangle. If it fits the triangle model better, the corresponding capsule location is determined to be the transverse colon. Otherwise, the corresponding capsule location is determined to be in the ascending/descending colon. Furthermore, if the observed shape changes from a circle to a triangle, this implies that the capsule is entering the transverse colon from the ascending colon. If the observed shape changes from a triangle to a circle, this implies that the capsule is entering the descending colon from the transverse colon.

In a real-world situation, the lumen wall is neither a perfect triangular shape nor a perfect circular shape. In order to develop a reliable method to differentiate the lumen wall shape, a frequency-domain method is disclosed. According to one embodiment of the present invention, the data series corresponding to the distances at various polar angles (as shown in Figs. 5B, 5D, 6B, 6D and 6F) are determined. For example, if the data series contains N data and the N data are taken uniformly over 360°, two neighboring data are 360°/N apart. For example, if N is equal to 180, the distance data is determined every 2°. In this case, $a_0$ is the distance at 0°, $a_1$ is the distance at 2°, $a_2$ is the distance at 4°,..., and $a_{(180-1)}$ is the distance at 358°. According to one embodiment of the present invention, the data series $a_0$, $a_1$, $a_2$,..., $a_{(N-1)}$ are converted to transform domain data. Any discrete transform, such as Discrete Fourier Transform (DFT) can be used to obtain the transform domain data. The DFT, $A_0$, $A_1$, $A_2$,..., $A_{(N-1)}$ for the input data, i.e., the data series $a_0$, $a_1$, $a_2$,..., $a_{(N-1)}$ can be derived from the input data, i.e., the data series $a_0$, $a_1$, $a_2$,..., $a_{(N-1)}$, according to:

$$A_k = \sum_{n=0}^{(N-1)} a_n \, e^{-\frac{2\pi i}{N} kn}, \; k=0,...,(N-1) \tag{1}$$

The input data can be recovered by applying inverse DFT (IDFT) to the frequency-domain data, $A_0$, $A_1$, $A_2$,..., $A_{(N-1)}$:

$$a_n = \frac{1}{N} \sum_{k=0}^{(N-1)} A_n \, e^{\frac{2\pi i}{N}kn}, \, n=0,\ldots,(N\text{-}1) \tag{2}$$

The term $A_0$ is also referred as the DC term since $A_0 = (a_0 + a_1 + a_2 + \ldots + a_{(N-1)})$. In other words, $A_0$ corresponds to the sum of input data. In this disclosure, $A_i$ is referred as the i-th frequency term, where i = 1, ..., (N-1). It is well known in the field of digital signal processing that Fast Fourier Transform (FFT) is often applied when N is equal to a power of 2 number such as 128, 256, 512, etc. In this case, computationally efficient algorithms can be used to substantially reduce the required computations for forward and inverse DFT. The present invention can also be benefitted from the FFT.

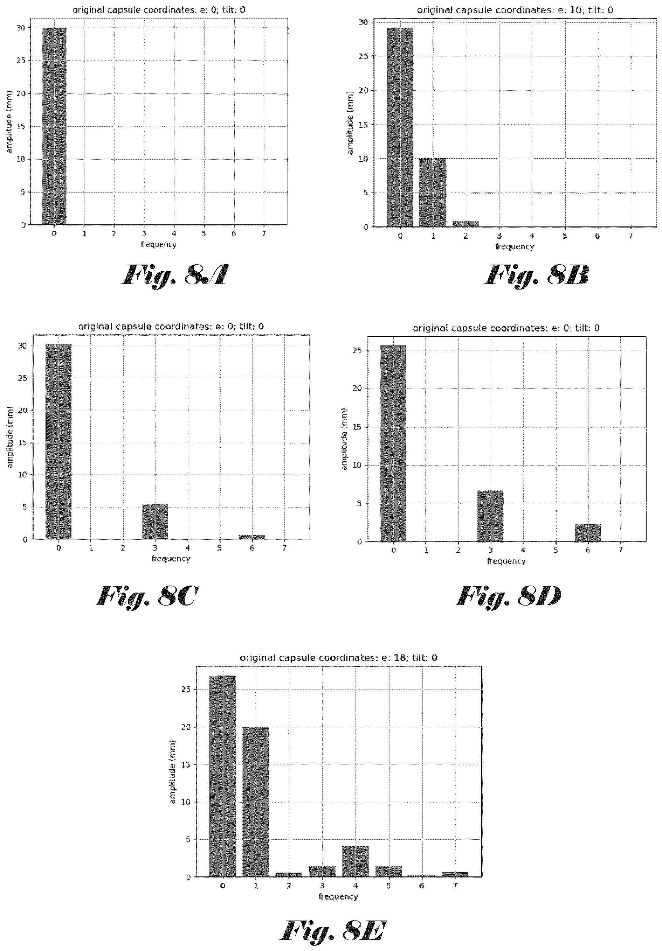

The amplitudes of the lower frequency terms of the frequency-domain data are shown in Figs. 8A-E for the centered circle case of Fig. 5A, the circle with eccentricity case of Fig. 5C, the centered triangle case of Fig. 6A, the centered triangle with sharper corners case of Fig. 6C, and the triangle with eccentricity case of Fig. 6E respectively. For the case of a centered circle of Fig. 5A, only the DC term, i.e., $A_0$, is non-zero and all the other frequency terms are zero as shown in Fig. 8A. When there is eccentricity in the case of a circle, the frequency domain still shows a dominant frequency term at $A_0$ as shown in Fig. 8B. However, there is also a notable frequency term at $A_1$ and a small frequency term at $A_2$ as shown in Fig. 8B. All other terms are negligible since their magnitudes are extremely small. Based on Fig. 8A and Fig. 8B, the effect of eccentricity is to cause some additional frequency terms, in particular $A_1$ and $A_2$. When the eccentricity is small (denoted as $E$), the $A_0$ in Fig. 8B can be derived as $A_0 = R - E^2/4R$, where $R$ is the radius of the circle. When $E$ is equal to 0 (i.e., no eccentricity), $A_0$ becomes R as expected. Furthermore, $A_1$ can be derived as $A_1 = E$ and $A_2$ can be derived as $A_2 = E^2/4R$. Therefore, larger eccentricity will cause larger $A_1$ and $A_2$.

In Fig. 8C, the amplitudes of the lower frequency terms are shown for a centered triangle, where a peak value exists at $A_0$. However, there is also a notable term at $A_3$ and a small term at $A_6$. In Fig. 8D, the amplitudes of the lower frequency terms are shown for a centered triangle with sharper corners, where a peak value exists at $A_0$. However, there is also a more notable term at $A_3$ and a small term at $A_6$. Compared with Fig. 8C, the frequency terms $A_3$ and $A_6$ are higher for Fig. 8D. In other words, sharper corners cause larger amplitudes in $A_3$ and $A_6$. In an extreme case where three corners are fully rounded as sections of a circle (i.e., the triangle becomes a circle), $A_3$ and $A_6$ become zero as expected. In the case of a triangle with eccentricity (as shown in Fig. 6E), the frequency terms are shown in Fig. 8E, where in addition to $A_0$, $A_1$ also has significant amplitude. As shown in Fig. 8E, there are also a few noticeable frequency terms in the low frequency region (e.g., $A_2$ to $A_7$).

As evidenced in Figs. 8C and 8D, the centered triangular shape always has a notable frequency term at $A_3$ in additional to $A_0$. For a centered circle case, only $A_0$ term exists. Therefore, for the cases of centered circle and centered triangle, the frequency domain signal can provide a clean indication as being a circle or a triangle. In particular, the $A_3$ term can be a signature for the centered triangle shape. Therefore, checking the amplitude of $A_3$ will provide a good indication regarding whether an underlying shape is triangular or not. In one embodiment, the magnitude ratio of $A_3$ and $A_0$ is compared with a threshold. For example, the threshold includes a range from 0.13 to 0.27. If the magnitude ratio of $A_3$ and $A_0$ is greater than the threshold, the shape is determined to be a triangle. Otherwise, the shape is determined to be a circle.

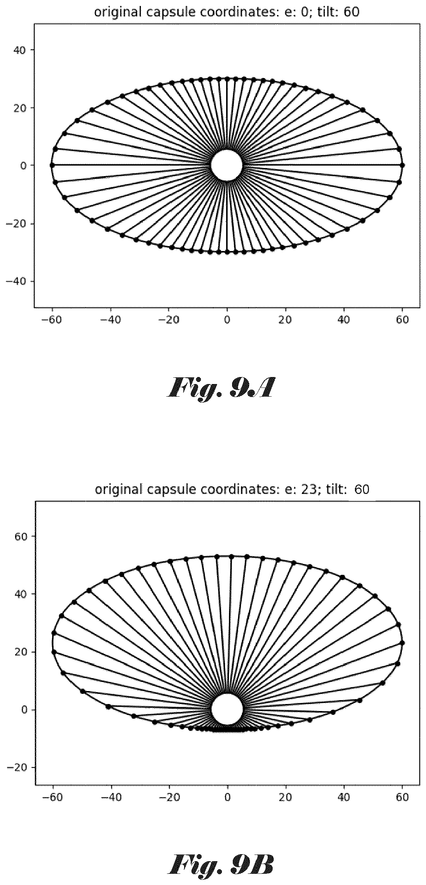

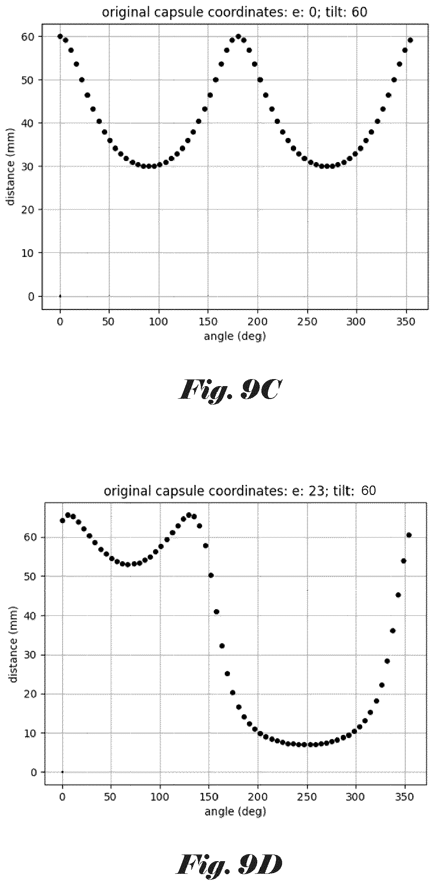

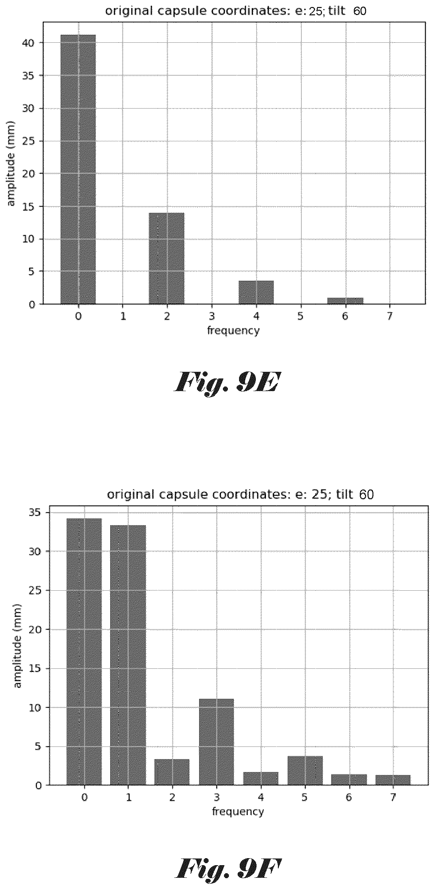

Furthermore, when the capsule travels in the GI tract, it is almost impossible to keep the longitudinal axis of the capsule always aligned with the longitudinal direction of the GI tract. Therefore, tilting often exists when the capsule travels through the GI tract. When a circular model is used for the GI tract, the intersection of a measuring cone and the lumen wall has an elliptical shape instead of the circular shape when tilting exists. Fig. 9A illustrates an example of distances from the capsule to the circular lumen wall with tilting, where the capsule is tilted by about 60° from the longitudinal direction of the GI tract. As shown in Fig. 9A, the shape of the intersection of the lumen wall and a measuring cone in the case of tilting becomes an ellipse. Fig. 9B shows an example of tilting with eccentricity. The distance plots in the polar coordinate are shown in Fig. 9C and Fig. 9D for Fig. 9A and Fig. 9B respectively. The frequency response corresponding to Fig. 9A is shown in Fig. 9E, where the frequency response shows substantial frequency terms at $A_2$ (roughly over 30% of $A_0$) and $A_4$ (roughly 10% of $A_0$). Also, there is a small response at $A_6$ (roughly less than 5% of $A_0$). The frequency terms at $A_1, A_3, A_5$, etc. are negligible. Compared to Fig. 8A for the case without tilting, the effect of tilting causes some frequency response at frequency terms other than $A_0$ (i.e., $A_2, A_4, A_6$, etc.). While the tilting results in some noticeable frequency terms in the low frequency region, the signal at $A_3$ or its harmonics is very minimal. Therefore, tilting alone in the case of circular colon model doesn't seem to cause misclassification as a triangular colon. The frequency response corresponding to Fig. 9B is shown in Fig. 9F, where the amplitude of $A_1$ is almost as high as $A_0$. $A_3$ is also very sizeable (roughly 30% of $A_0$'s amplitude). Furthermore, there are also noticeable amplitudes for other lower frequency terms as shown in Fig. 9F. Since there is a sizeable signal strength at $A_3$, it may be hard to distinguish whether it is a circle or a triangle.

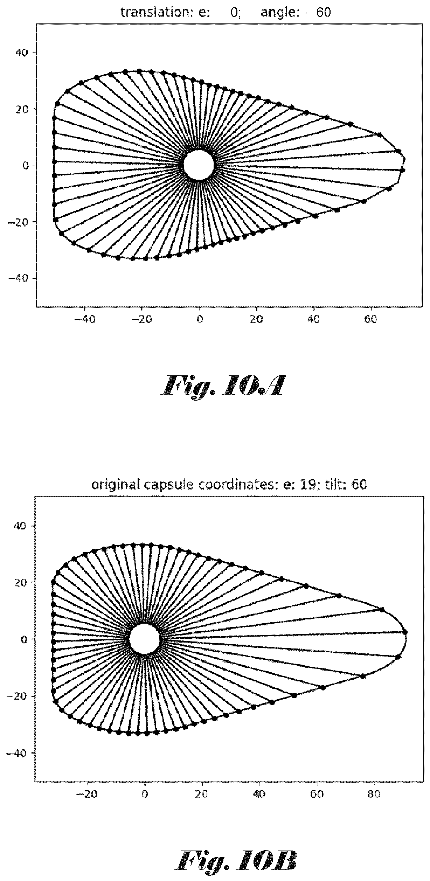

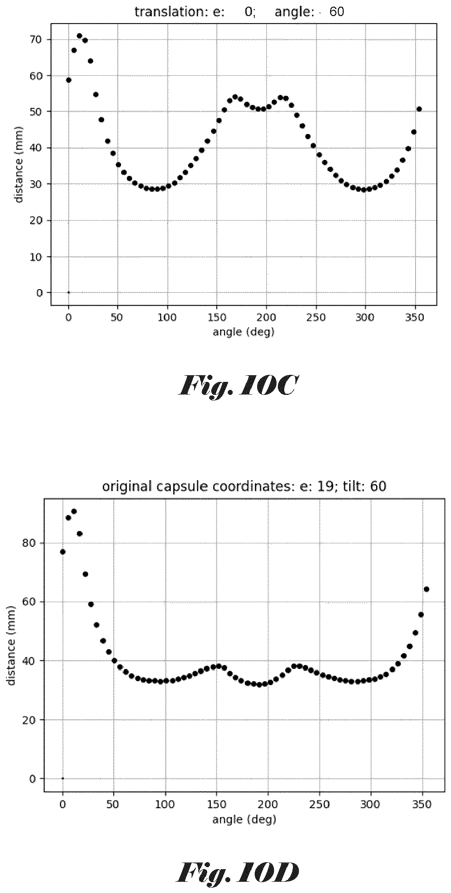

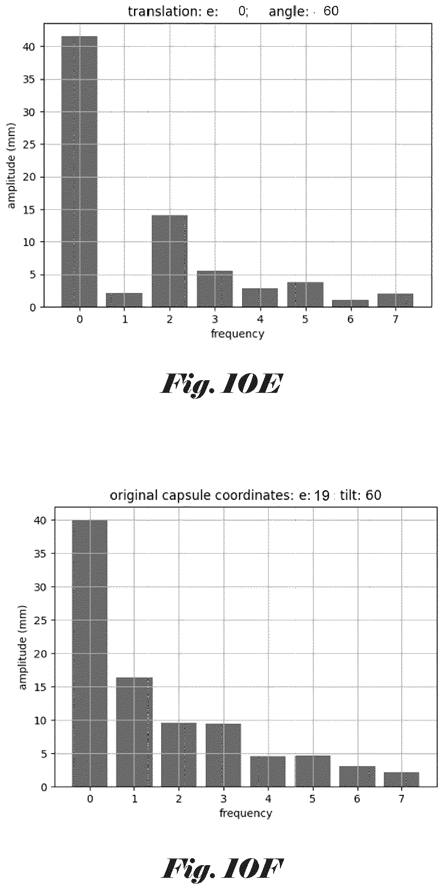

When a triangular model is used for the GI tract, the intersection of a measuring cone and the lumen wall will not have an equilateral shape anymore when tilting exists. Fig. 10A illustrates an example of distances from the capsule to the triangular lumen wall with tilting, where the capsule is tilted by about 60° from the longitudinal direction of the GI tract. As shown in Fig. 10A, the shape of the intersection of the lumen wall and a measuring cone in the case of tilting becomes an isosceles triangle. While the example in Fig. 10A shows an isosceles triangle due to tilting, the tilting may result in an arbitrary triangle. Fig. 10B shows an example of tilting with eccentricity. The distance plots in the polar coordinate are shown in Fig. 10C and Fig. 10D for Fig. 10A and Fig. 10B respectively. The frequency response corresponding to Fig. 10A is shown in Fig. 10E, where the frequency response shows noticeable frequency terms at $A_1$, $A_2$, $A_4$, $A_5$ and $A_7$ in additional *to* $A_3$ and $A_6$. Compared to Fig. 8C for the case without tilting, the effect of tilting causes some frequency response at frequency terms (i.e., $A_1$, $A_2$, $A_4$, $A_5$ and $A_7$, etc.). In this case, the tilting results in some noticeable frequency terms in the low frequency region, which may affect correct segmentation. The frequency response corresponding to Fig. 10B is shown in Fig. 10F, where the low frequency terms other than $A_3$ and $A_6$ become more prominent, which may further affect correct segmentation.

As illustrated above, the frequency domain characteristics between a centered circle and a centered triangle without tilting are very distinct. When tilting and/or eccentricity occurs, the distinction in the frequency domain characteristics becomes less clear, which may affect the correctness of frequency-domain based segmentation. In order to overcome this issue, an embodiment of the present invention corrects the eccentricity and/or tilting before applying the DFT. In order to offset the eccentricity, a method of coordinate translation is disclosed. In the coordinate translation process, we need to find the centroid of the 2D contour (e.g., the contours in Fig. 5C and Fig. 6E). In the (x,y) coordinate, the locations (($x_i, y_i$), $i$=0,...,($N$-1)) of the dots corresponding to the ending point of the measuring rays can be determined. The image moments $M_{pg}$ for the image associated with the dots (($x_i, y_i$) are defined as:

$$M_{pq} = \sum_{i=0}^{N-1} \sum_{j=0}^{N-1} x_i^p \, y_j^q. \tag{3}$$

The centroid $\{\bar{x}, \bar{y}\}$ can then be derived as:

$$\{\bar{x}, \bar{y}\} = \left\{ \frac{M_{10}}{M_{00}} \frac{M_{01}}{M_{00}} \right\}. \tag{4}$$

Upon the determination of the centroid $\{\bar{x}, \bar{y}\}$, the contour for the lumen wall can be shifted so that the capsule is equivalently located in the virtual center of the lumen wall.

In yet another embodiment of the present invention, the tilting can be corrected by coordinate rotation. For the coordinate rotation process, the central moments $\mu_{pq}$ of the image is first derived according to:

$$\mu_{pq} = \sum_{i=0}^{N-1} \sum_{j=0}^{N-1} (x_i - \bar{x})^p \, (y_j - \bar{y})^q. \tag{5}$$

Furthermore, a set of central moments are determined as follows:

$$\mu'_{20} = \frac{\mu_{20}}{\mu_{00}} = \frac{M_{20}}{M_{00}} - \bar{x}^2 \tag{6}$$

$$\mu'_{02} = \frac{\mu_{02}}{\mu_{00}} = \frac{M_{02}}{M_{00}} - \bar{y}^2 \tag{7}$$

$$\mu'_{11} = \frac{\mu_{11}}{\mu_{00}} = \frac{M_{11}}{M_{00}} - \bar{x}\bar{y} \tag{8}$$

A covariance matrix $\begin{bmatrix} \mu'_{20} & \mu'_{11} \\ \mu'_{11} & \mu'_{02} \end{bmatrix}$ is then defined accordingly. The eigenvectors of this matrix correspond to the major and minor axes of the image intensity. The tilting angle can be determined according to:

$$cos\varphi = \sqrt{\frac{I_b}{I_a}}.$$ (9)

In the above equation, $I_a$ and $I_b$ correspond to the eigenvalues of the major axis and the minor axis respectively.

The coordinate translation and coordinate rotation processes mentioned above can be applied to the distance information to offset the eccentricity and correct the tilting. After the coordinate translation and coordinate rotation processes, the eccentricity and tilting should be removed or substantially reduced. Therefore, the eccentricity/tilting corrected distance information should provide more reliable segmentation. The coordinate translation and coordinate rotation processes can be performed by applying the coordinate translation first followed by the coordinate rotation. Alternatively, the coordinate rotation can be applied first and followed by the coordinate translation. Furthermore, a system may choose to apply only one of these processes. For example, a system may apply coordinate translation without coordinate rotation. In another example, the system may choose to use coordinate rotation without coordinate translation.

Fig. 5C illustrates an example of eccentricity without tilting for the circular GI tract. The coordinate translation process mentioned above can be applied to the distance information to correct the eccentricity. In other words, the process as described in equations (3) and (4) can be applied to the distance information to determine the centroid. After the centroid is obtained, the distance information as represented in the (x,y)-coordinate can be offset by the derived centroid. After the coordinate translation process, the eccentricity-offset distance information becomes centered as shown in Fig. 5A.

Fig. 6E illustrates an example of eccentricity without tilting for the triangular GI tract. The coordinate translation process mentioned above can be applied to the distance information. After the coordinate translation process, the eccentricity-offset distance information becomes centered as shown in Fig. 6A.

As shown in Figs. 8A and 8C, the characteristics of frequency response between a centered circle and a centered triangle without tilting are very distinct. Therefore, the distance information of Fig. 5C becomes a centered circle as shown in Fig. 5A and the distance information of Fig. 6E becomes a centered triangle as shown in Fig. 6A after the coordinate translation process. As shown in Figs. 8A and 8C, the frequency-domain characteristics of the centered circle and the centered triangle are very distinct. Thus, the segmentation can be performed reliably.

Fig. 9A illustrates an example of a centered capsule with tilting for the circular GI tract. The coordinate rotation process mentioned above can be applied to the distance information (i.e., an ellipse) to correct the tilting. In other words, the process as described in equations (3)-(9) can be applied to the distance information to determine the tilting angle. After the tilting angle is obtained, the distance information as represented in the (x,y)-coordinate can be rotated by the derived tilting angle. After the coordinate rotation process, the rotation-corrected distance information becomes a circle as shown in Fig. 5A.

Fig. 10A illustrates an example of a centered capsule with tilting for the triangular GI tract. The coordinate rotation process mentioned above can be applied to the distance information. After the coordinate rotation process, the rotation-corrected distance information becomes a circle as shown in Fig. 6A.

As shown in Figs. 8A and 8C, the characteristics of frequency response between a centered circle and a centered triangle without tilting are very distinct. Therefore, the distance information of Fig. 9A is processed by coordinate rotation to result in a centered circle without tilting as shown in Fig. 5A and the distance information of Fig. 10A is processed by coordinate rotation to result in a centered triangle without tilting as shown in Fig. 6A. the frequency-domain characteristics of the centered circle and the centered triangle are very distinct. Thus, the segmentation can be performed reliably.

Fig. 9B illustrates an example of eccentricity with tilting for the circular GI tract. The coordinate rotation process mentioned above can be applied to the distance information. After the coordinate rotation process, the rotation-corrected distance information is only affected by eccentricity as shown in Fig. 5C. The coordinate translation process mentioned above can be further applied to the processed distance information. After the coordinate translation process, the further processed distance information becomes centered as shown in Fig. 5A. While the coordinate rotation is applied and followed by the coordinate translation in the above example, the order of the two processes can also be swapped, i.e., the coordinate translation first followed by the coordinate rotation. In this case, the processed distance information after the first step is shown in Fig. 9B. After the coordinate rotation is applied, the processed distance information after the second step is shown in Fig. 5A.

Fig. 10B illustrates an example of eccentricity with tilting for the triangular GI tract. The coordinate rotation process mentioned above can be applied to the distance information. After the coordinate rotation process, the rotation-corrected distance information is only affected by eccentricity as shown in Fig. 6E. The coordinate translation process mentioned above can be further applied to the processed distance information. After the coordinate translation process, the further processed distance information becomes centered as shown in Fig. 6A. While the coordinate rotation is applied and followed by the coordinate translation in the above example, the order of the two processes can also be swapped, i.e., coordinate translation first followed by coordinate rotation. In this case, the processed distance information after the first step is shown in Fig. 10A. After the coordinate rotation is applied, the processed distance information after the second step is shown in Fig. 6A.

As shown in Figs. 8A and 8C, the characteristics of frequency response between a centered circle and a centered triangle without tilting are very distinct. Therefore, the distance information of Fig. 9B is processed by coordinate translation and coordinate rotation to result in a centered circle without tilting as shown in Fig. 5A and the distance information of Fig. 10B is processed by coordinate translation and coordinate rotation to result in a centered triangle without tilting as shown in Fig. 6A. The frequency-domain characteristics of the centered circle and the centered triangle are very distinct. Thus, the segmentation can be performed reliably.

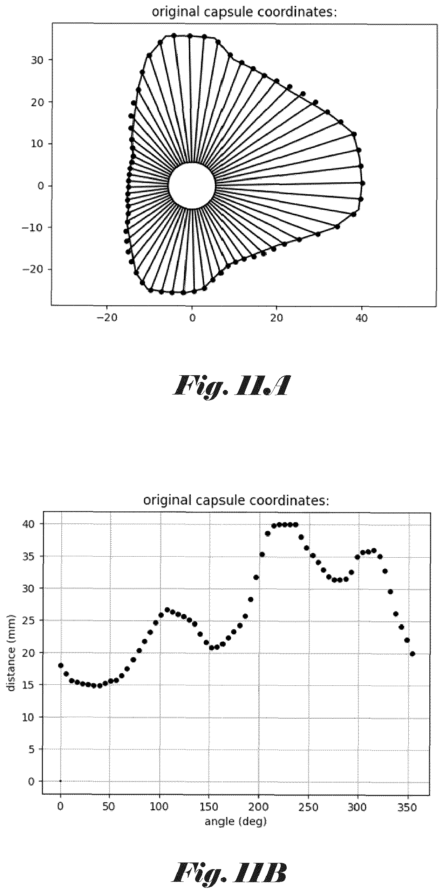

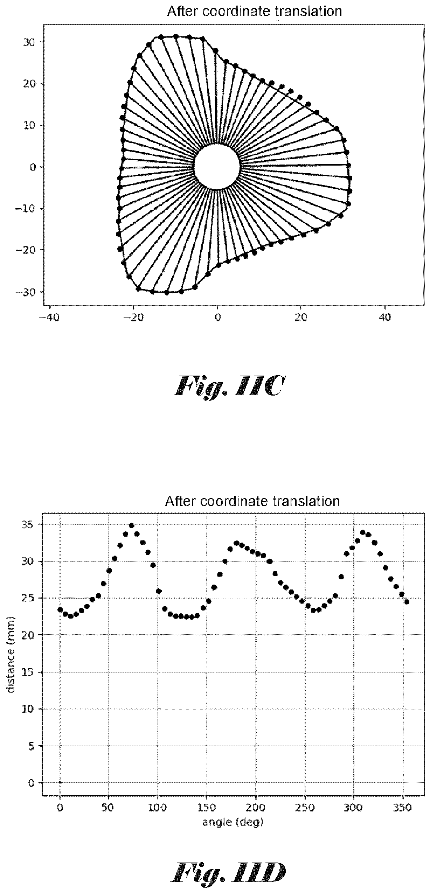

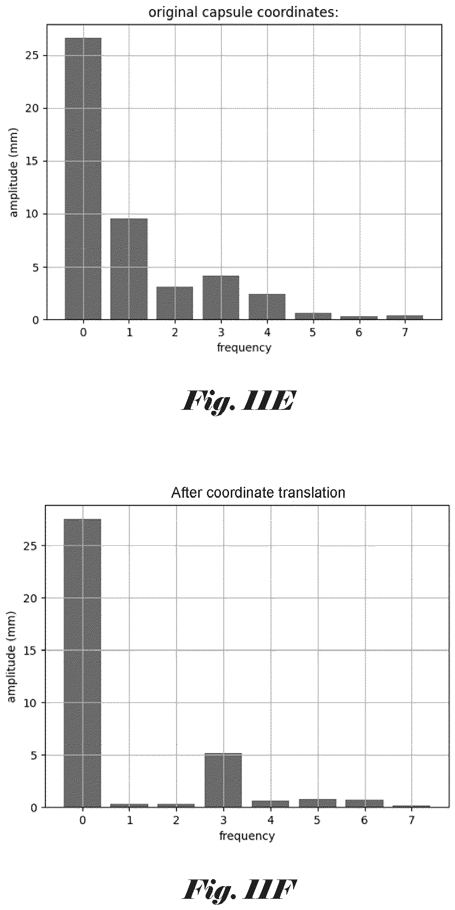

In reality, the shape of the lumen wall of the colon is never a perfect circle nor a perfect triangle. It is expected that there will be some variations between an actual lumen wall and the ideal model. The method of eccentricity correction has been applied to a set of measured distance data in-vitro. Fig. 11A illustrates an example of distances from the capsule to the lumen wall of the transverse colon in an in-vitro environment, where the capsule is off center without tilting. As shown in Fig. 11A, the transverse colon wall has a triangular shape. Fig. 11B illustrates the corresponding distances in the polar coordinate for Fig. 11A. As mentioned earlier, the centroid can be determined according to equations (3) and (4). The eccentricity can be corrected accordingly. Fig. 11C illustrates the distances for Fig. 11A after eccentricity correction according to an embodiment of the present invention. Fig. 11D illustrates the distances in the polar coordinate for Fig. 11C. Fig. 11E illustrates the amplitude of the lower frequency terms of the frequency response for Fig. 11A. As shown in Fig. 11E, the eccentricity causes significant frequency terms at $A_1$, $A_2$ and $A_4$. Fig. 11F illustrates the amplitude of the lower frequency terms of the frequency response for Fig. 11C. After eccentricity correction, frequency terms at $A_1$, $A_2$ and $A_4$ are substantially suppressed while frequency term at $A_3$ becomes larger. Based on the frequency response in Fig. 11F, the characteristics of a triangular shape becomes very obvious.

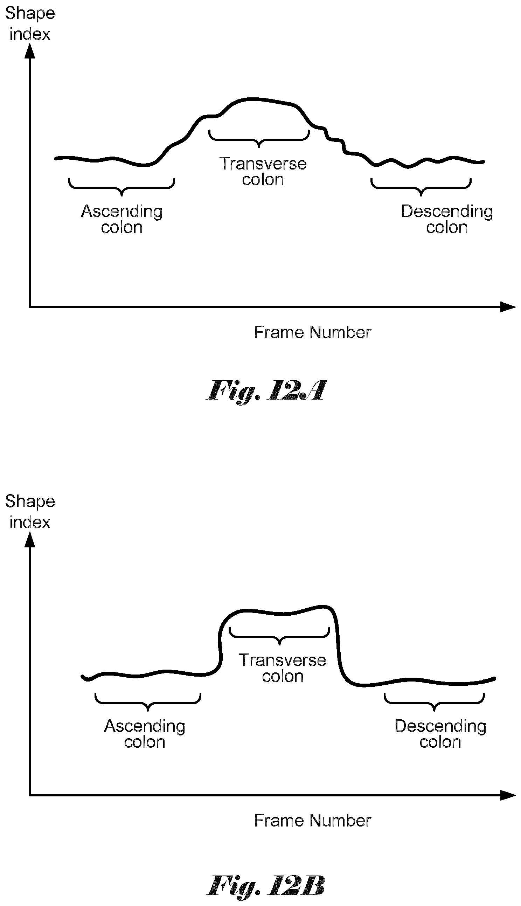

In another embodiment, we may utilize a derivative of the distance information for determining the segmentation of the colon associated with the capsule location. For example, we may use the lumen size (i.e., the y axis) as a function of time (i.e., the x axis) and determine the segmentation based on the derivative of the lumen size function. In another embodiment, the frame number could be used for the x axis. In another embodiment, an index of the shape is used to indicate that the capsule is in ascending colon, transverse colon, descending colon or more distant colon based on the index. For example, the index of the shape may be based on the frequency transformation of the geometry derived from distance information. For example, the frequency transformation corresponds to a ratio of the third frequency-term magnitude and the zero-th frequency-term magnitude. Two examples of the shape index based segmentation are shown in Figs. 12A-B, where the ascending colon, transverse colon and descending colon can be discerned from the shape index. Since the distance information or its derivative varies from frame to frame, a smoothed distance information may be more reliable. For example, the smoothed distance information may be derived as a moving average of every N frames or a filtered information can be used to draw the plot.

In another embodiment, the travelled distance of the capsule device can be used as the x-axis for the plots (e.g., distance, shape, shape index or frequency information) instead of the frame number or the frame time. A method of measuring the travelled distance of the capsule device has been disclosed in U.S. Patent No. 10,506,921, issued on Dec. 17, 2019. According to U.S. Patent No. 10,506,921, the motion vectors can be used to derive the travelled distance of the capsule device in the longitudinal direction of the GI tract. Furthermore, U.S. Patent No. 10,506,921 also discloses a method to refine or adjust the travelled distance based on the distance information between the capsule device and the surrounding lumen wall. Therefore, the anatomical geometry information along the colon longitudinal direction can be assessed by a GI doctor before the colonoscopy procedure is performed to remove the polyp to make the procedure with less efforts while enhancing safety.

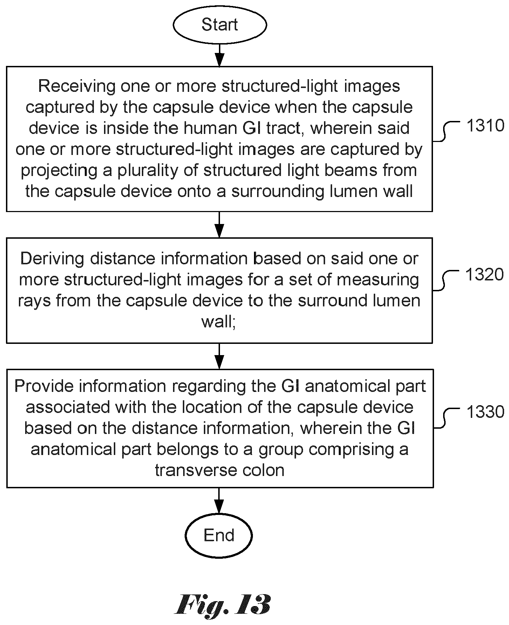

Fig. 13 illustrates an exemplary flowchart for identifying a GI (gastrointestinal) part associated with a location of a capsule device within a human GI tract according to an embodiment of the present invention. According to this method, one or more structured-light images captured by the capsule device when the capsule device is inside the human GI tract are received in step 1310, where said one or more structured-light images are captured by projecting a plurality of structured light beams from the capsule device onto a surrounding lumen wall. The structured-light images may be captured along with regular images so that the shape/size information derived from the structured-light image may be used by the regular images. Distance information is derived based on said one or more structured-light images for a set of measuring rays from the capsule device to the surround lumen wall in step 1320. Information regarding the GI part associated with the location of the capsule device is provided based on the distance information in step 1330, wherein the GI part belongs to a group comprising a transverse colon. The GI part associated with the location of the capsule device may provide useful information for the corresponding regular images. For example, if a polyp is found in a regular image, the location of the capsule device (presumably the regular image with the polyp is captured at this location) can be used to identify the location of the polyp.

The method for identifying a GI (gastrointestinal) part associated with the location of the capsule device within the human GI tract can be implemented using one or more electronic circuits or processors. Furthermore, said one or more electronic circuits or processors can be programmable according to software or firmware. Said one or more electronic circuits or processors can be embedded within the capsule device and perform all the processing steps mentioned above. In this case, the location of the capsule device within the human GI tract can be determined in real-time or with a small processing delay after one or more structured-light images are received. The fully embedded approach will consume more precious battery power of the capsule device. However, the capsule device may take advantage of the real-time or near real-time location information and perform certain task adaptively. For example, the capsule device may incorporate a camera to capture regular images of the lumen wall. The capture rate may be different for the transverse colon and ascending/descending colon.

In another embodiment, the processing steps mentioned above may be performed by one or more electronic circuits or processors external to the capsule device. For example, the structured-light images may be transmitted by a radio-frequency transmitter inside the capsule device to an external radio-frequency receiver. One or more electronic circuits or processors, a laptop, a computer or a workstation may be used to derive the location of the capsule device within the human GI tract. The location of the capsule device within the human GI tract can be correlated with regular images captured by the capsule device to determine the corresponding GI part associated with the regular images. For example, if a regular image contains abnormality (e.g., a polyp), a medical professional will be able to determine which part of the GI tract (e.g., descending color) that the abnormality is located. In another example, the capsule device may have on-board storage to store the captured regular images and structured images. Upon excretion from the human body, the capsule device can be retrieved and the stored images can be down loaded. The processing steps mentioned above can be performed by one or more electronic circuits or processors, a laptop, a computer or a workstation according to one embodiment of the present invention.

The above description is presented to enable a person of ordinary skill in the art to practice the present invention as provided in the context of a particular application and its requirements. Various modifications to the described embodiments will be apparent to those with skill in the art, and the general principles defined herein may be applied to other embodiments. Therefore, the present invention is not intended to be limited to the particular embodiments shown and described, but is to be accorded the widest scope consistent with the principles and novel features herein disclosed. In the above detailed description, various specific details are illustrated in order to provide a thorough understanding of the present invention. Nevertheless, it will be understood by those skilled in the art that the present invention may be practiced.

The invention may be embodied in other specific forms without departing from its spirit or essential characteristics. The described examples are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A method of identifying a colon anatomical part associated with a location of a capsule device within a human colon, **characterized in that** the method comprises:

   receiving one or more structured-light images captured by the capsule device when the capsule device is inside the human colon, wherein said one or more structured-light images are captured by projecting a plurality of structured light beams from the capsule device onto a surrounding lumen wall;
   deriving distance information based on said one or more structured-light images for a target set of measuring rays from the capsule device to the surrounding lumen wall; and
   providing information regarding the colon anatomical part associated with the location of the capsule device associated with a distance-measuring frame related to said one or more structured-light images based on the distance information, wherein the colon anatomical part belongs to a group comprising a transverse colon.

2. The method of Claim 1, wherein the target set of measuring rays are on a plane perpendicular to a longitudinal axis of the capsule device and each of the measuring rays ends at an intersection of the plane and an interior point of the surrounding lumen wall.

3. The method of Claim 2, further comprising displaying visual representation of ending points of the measuring rays in a Cartesian coordinate on a display device.

4. The method of Claim 1, further comprising providing information indicative of a shape of the surrounding lumen wall based on the distance information, wherein the location of the capsule device is indicated according to whether the shape of the surrounding lumen wall resembles a circle or a triangle.

**5.** The method of Claim 4, wherein curve fitting is used to indicate whether the shape of the surrounding lumen wall resembles the triangle; or further comprising displaying a plot on a display device for a user to visualize, wherein the plot is representative of the shape of the surrounding lumen wall in one axis and a travelled distance of the capsule device in another axis; or further comprising displaying a plot on a display device for a user to visualize, wherein the plot is representative of the shape of the surrounding lumen wall in one axis and a frame number or a frame time in another axis, preferably wherein the colon anatomical part associated with the location of the capsule device is indicated according to the shape of the surrounding lumen wall, and wherein if the shape resembles the triangle, the colon anatomical part associated with the location of the capsule device is the transverse colon; or further comprising displaying a plot on a display device for a user to visualize, wherein the plot is representative of a shape index of the surrounding lumen wall in one axis and a frame number or a frame time in another axis, and wherein the shape index is derived based on frequency-domain data of the distance information represented in a polar coordinate, preferably further comprising displaying a plot on a display device for a user to visualize, wherein the plot is representative of the shape index of the surrounding lumen wall in one axis and a travelled distance of the capsule device in another axis, and particularly wherein the colon anatomical part associated with the location of the capsule device is indicated according to the shape index and if the shape index indicates the triangle, the colon anatomical part associated with the location of the capsule device is the transverse colon.

**6.** The method of Claim 1, further comprising determining a size of the surrounding lumen wall based on the distance information between the capsule device and the surrounding lumen wall.

**7.** The method of Claim 6, further comprising displaying a plot on a display device for a user to visualize, wherein the plot is representative of the size of the surrounding lumen wall in one axis and a travelled distance of the capsule device in another axis; or further comprising displaying a plot on a display device for a user to visualize, wherein the plot is representative of the size of the surrounding lumen wall in one axis and a frame number or a frame time in another axis, preferably wherein the colon anatomical part associated with the location of the capsule device is indicated according to the size or a trend of the size of the surrounding lumen wall, more preferably wherein a section of the human colon corresponds to an ascending colon if the section corresponds to a first part of the human colon and the size is reduced or the size is gradually reducing, preferably wherein the size of the surrounding lumen wall is determined based on the distance information associated with multiple distance-measuring frames close to each other in a temporal order, more preferably wherein the colon anatomical part associated with the location of the capsule device is indicated based on the distance information associated with the multiple distance-measuring frames, and a decision of the colon anatomical part associated with the location of the capsule device is confirmed when a same decision out of multiple consecutive decisions is reached, and particularly wherein the plot representative of the size of the surrounding lumen wall corresponds an average or filtered size of the surrounding lumen wall associated with multiple measuring frames.

**8.** The method of Claim 1, further comprising transforming the distance information represented in a polar coordinate into discrete frequency-domain information, wherein the colon anatomical part associated with the location of the capsule device is indicated by the discrete frequency-domain information.

**9.** The method of Claim 8, wherein Discrete Fourier Transform (DFT) or Fast Fourier Transform (FFT) is used to transform the distance information into the discrete frequency-domain information; or wherein if all frequency terms are insignificant except for zero-th frequency term, the colon anatomical part associated with the location of the capsule device corresponds to ascending or descending colon; or wherein if the discrete frequency-domain information has a maximum at zero-th frequency term of the discrete frequency-domain information and a second largest term at third frequency term of the discrete frequency-domain information, the colon anatomical part associated with the location of the capsule device corresponds to the transverse colon; or further comprising determining a magnitude ratio of zero-th frequency term and third frequency term of the discrete frequency-domain information, wherein if ratio is larger than a threshold, the colon anatomical part associated with the location of the capsule device corresponds to the transverse colon and, otherwise the colon anatomical part associated with the location of the capsule device corresponds to ascending/descending colon, and wherein the threshold includes a range between 0.13 and 0.27; or further comprising applying coordinate translation to the distance information represented in the polar coordinate to correct eccentricity prior to said transforming the distance information into the discrete frequency-domain information; or further comprising applying coordinate rotation to the distance information represented in the polar coordinate to correct tilting prior to said transforming the distance information into the discrete frequency-domain information; or further comprising applying coordinate translation to the distance information represented in the polar coordinate to correct eccentricity following by applying coordinate rotation to the distance information to correct tilting prior to said transforming the distance information into the discrete frequency-domain information;

or further comprising applying coordinate rotation to the distance information represented in the polar coordinate to correct tilting following by applying coordinate translation to the distance information to correct eccentricity prior to said transforming the distance information into the discrete frequency-domain information.

10. The method of Claim 1, further comprising receiving a regular image capsuled by a camera in the capsule device for a scene in a field view of the camera and determining an anomaly in the regular image, wherein the regular image is temporally close to said one or more structured-light images and the colon anatomical part associated with the location of the anomaly within the human colon is indicated based on the distance information; or further comprising displaying a plot on a display device for a user to visualize, wherein the plot is representative of discrete frequency-domain data in one axis and a travelled distance of the capsule device in another axis, wherein the discrete frequency-domain data are derived by applying frequency transformation to the distance information represented in a polar coordinate.

11. The method of Claim 1, further comprising determining fold depth information of the surrounding lumen wall based on the distance information.

12. The method of Claim 11, further comprising displaying a plot on a display device for a user to visualize, wherein the plot is representative of the fold depth information of the surrounding lumen wall in one axis and a travelled distance of the capsule device in another axis; or further comprising displaying a plot on a display device for a user to visualize, wherein the plot is representative of the fold depth information of the surrounding lumen wall in one axis and a frame number or a frame time in another axis, preferably wherein the colon anatomical part associated with the location of an anomaly within the human colon is indicated based on the fold depth information.

13. The method of Claim 1, further comprising displaying a plot on a display device for a user to visualize, wherein the plot is representative of discrete frequency-domain data in one axis and a frame number or a frame time in another axis, wherein the discrete frequency-domain data are derived by applying frequency transformation to the distance information represented in a polar coordinate.

14. The method of Claim 13, wherein the discrete frequency-domain data corresponds to a ratio of a magnitude of third frequency term to a magnitude of zero-th frequency term.

15. A system for identifying a colon anatomical part associated with a location of a capsule device within a human colon, **characterized in that** the system comprises one or more electronic circuits or processors configured to:

    receive one or more structured-light images captured by the capsule device when the capsule device is inside the human colon, wherein said one or more structured-light images are captured by projecting a plurality of structured light beams from the capsule device onto a surrounding lumen wall;
    derive distance information based on said one or more structured-light images for a target set of measuring rays from the capsule device to the surrounding lumen wall; and
    provide information regarding the colon anatomical part associated with the location of the capsule device based on the distance information, wherein the colon anatomical part belongs to a group comprising a transverse colon.

*Fig. 1*

*Fig. 2*

**Fig. 3**

**Fig. 4**

original capsule coordinates: e: 0; tilt: 0

530
532
534
510
520

## Fig. 5A

original capsule coordinates: e: 0; tilt: 0

distance (mm)

angle (deg)

## Fig. 5B

original capsule coordinates: e: 23; tilt: 0

**Fig. 5C**

original capsule coordinates: e: 23; tilt: 0

**Fig. 5D**

original capsule coordinates: e: 0; tilt: 0

610

620

## Fig. 6A

original capsule coordinates: e: 0; tilt: 0

## Fig. 6B

original capsule coordinates: e: 0; tilt: 0

630

640

## Fig. 6C

original capsule coordinates: e: 0; tilt: 0

distance (mm)

angle (deg)

## Fig. 6D

original capsule coordinates: e: 19; tilt: 0

620

610

*Fig. 6E*

original capsule coordinates: e: 19; tilt: 0

*Fig. 6F*

*Fig. 7A*

*Fig. 7B*

Fig. 8A

Fig. 8B

Fig. 8C

Fig. 8D

Fig. 8E

Fig. 9A

Fig. 9B

**Fig. 9C**

**Fig. 9D**

*Fig. 9E*

*Fig. 9F*

**Fig. 10A**

**Fig. 10B**

*Fig. 10C*

*Fig. 10D*

*Fig. 10E*

*Fig. 10F*

original capsule coordinates:

*Fig. 11A*

original capsule coordinates:

*Fig. 11B*

After coordinate translation

*Fig. IIC*

After coordinate translation

*Fig. IID*

original capsule coordinates:

*Fig. IIE*

After coordinate translation

*Fig. IIF*

*Fig. 12A*

*Fig. 12B*

Start

Receiving one or more structured-light images captured by the capsule device when the capsule device is inside the human GI tract, wherein said one or more structured-light images are captured by projecting a plurality of structured light beams from the capsule device onto a surrounding lumen wall — 1310

Deriving distance information based on said one or more structured-light images for a set of measuring rays from the capsule device to the surround lumen wall; — 1320

Provide information regarding the GI anatomical part associated with the location of the capsule device based on the distance information, wherein the GI anatomical part belongs to a group comprising a transverse colon — 1330

End

*Fig. 13*

**EUROPEAN SEARCH REPORT**

Europäisches Patentamt
European Patent Office
Office européen des brevets

Application Number

EP 23 17 7902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 506 921 B1 (WANG KANG-HUAI [US]) 17 December 2019 (2019-12-17) | 1,5-15 | INV. A61B1/00 A61B1/04 |
| Y | * column 7, line 6 - line 12; claim 12; figure 1 * | 2 | A61B1/06 A61B1/31 |
| X | US 10 102 334 B2 (RABINOVITZ ELISHA [IL]; BRAUN ORI [US] ET AL.) 16 October 2018 (2018-10-16) | 1,15 | |
| Y | * the whole document * | 3 | |
| X | US 2018/174318 A1 (WANG KANG-HUAI [US] ET AL) 21 June 2018 (2018-06-21) * paragraph [0018] * * paragraph [0023] - paragraph [0026] * * claim 5 * | 1,15 | |
| Y | US 7 465 271 B2 (HOYA CORP [JP]) 16 December 2008 (2008-12-16) * column 3, line 32 - line 43 * | 2,3 | |
| A | US 2021/267441 A1 (WANG KANG-HUAI [US] ET AL) 2 September 2021 (2021-09-02) * paragraph [0028] - paragraph [0036] * | 1-15 | **TECHNICAL FIELDS SEARCHED (IPC)** A61B |

The present search report has been drawn up for all claims

| Place of search | Date of completion of the search | Examiner |
|---|---|---|
| Munich | 26 October 2023 | Alvazzi Delfrate, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 292 507 A1

## ANNEX TO THE EUROPEAN SEARCH REPORT
## ON EUROPEAN PATENT APPLICATION NO.

EP 23 17 7902

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2023

| Patent document cited in search report | | Publication date | Patent family member(s) | | | Publication date |
|---|---|---|---|---|---|---|
| US 10506921 | B1 | 17-12-2019 | CN | 111035351 | A | 21-04-2020 |
| | | | CN | 114983317 | A | 02-09-2022 |
| | | | US | 10506921 | B1 | 17-12-2019 |
| | | | US | 2020113422 | A1 | 16-04-2020 |
| US 10102334 | B2 | 16-10-2018 | CN | 103370001 | A | 23-10-2013 |
| | | | US | 2013304446 | A1 | 14-11-2013 |
| | | | WO | 2012090197 | A1 | 05-07-2012 |
| US 2018174318 | A1 | 21-06-2018 | NONE | | | |
| US 7465271 | B2 | 16-12-2008 | DE | 102004042332 | A1 | 24-03-2005 |
| | | | JP | 2005074031 | A | 24-03-2005 |
| | | | US | 2005049462 | A1 | 03-03-2005 |
| US 2021267441 | A1 | 02-09-2021 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7817354 B
- US 7983458 B
- US 9936151 B
- US 10506921 B

- US 11019327 B
- US 10354382 B
- US 10102334 B